# EUROPEAN PATENT SPECIFICATION

(11) **EP 0 387 352 B1**
(45) Date of publication and mention of the grant of the patent: **13.07.1994**
(21) Application number: 89908863.7
(22) Date of filing: 26.07.1989
(51) Int. Cl.: A61K 37/02, A61K 37/26, A61K 47/00

(54) **ENTERIC FORMULATIONS OF PHYSIOLOGICALLY ACTIVE PEPTIDES AND PROTEINS**
ENTERISCHE FORMULIERUNGEN AUF BASIS PHYSIOLOGISCH AKTIVEN PEPTIDEN UND PROTEINEN
PREPARATIONS ENTERIQUES A BASE DE PROTEINES ET DE PEPTIDES PHYSIOLOGIQUEMENT ACTIFS

(30) Priority: 30.07.1988 JP 191185/88
(43) Date of publication of application: 19.09.1990
(73) Proprietor: TAKADA, Kanji, Shimogyo-ku, Kyoto-shi, Kyoto 600 (JP)
(72) Inventor: TAKADA, Kanji, Shimogyo-ku, Kyoto-shi, Kyoto 600 (JP)
(74) Representative: Kinzebach, Werner, Dr.
(86) International application number: JP8900748
(87) International publication number: WO9001329

(56) References cited:
- EP-A- 0 225 189
- Chemical Abstracts, vol. 107, no. 6, 10 Aug. 1987 (Columbus, Ohio, US), p. 420, abstract no. 46298j
- Chemical Abstracts, vol. 110, no. 20, 15 May 1989 (Columbus, Ohio,US), K. Takada et al., pp. 405-406, abstract no. 179441j
- Chemical Abstracts, vol. 96, no. 10, 8 March 1982 (Columbus, Ohio, US),p. 377, abstract no. 74626n

## Description

### Background of The Invention

This invention relates to a pharmaceutical formulation containing physiologically active peptides or proteins as an active principle.

A number of physiologically active peptides and proteins have been currently used for therapeutic purposes. Enzymatic degradation and poor absorption of these substances from the intestinal tract have historically limited their administration to the parenteral route. The blood level of a drug is a function of the rate of its uptake by the living body (bioavailability) and the rate of its removal from the body (clearance). In case of the oral administration of peptides or proteins, poor absorption from the intestinal tract is the main factor that precludes the dose-dependent delivery of the drug into the blood stream.

Attempts have been made to increase the intestinal absorption of proteinous drugs by the use of formulations that protect the drugs from enzymatic degradation and/or enhance the uptake into the intestinal mucosa. These include a combination of insulin and a chymotrypsin-inhibitor (J. Pharm. Pharmcol., 37 (1985), 545-549); coating with azoaromatic copolymers (Science, 233 (1986). 1081-1084); a combination of vasopressin and aprotinin as a proteolytic enzyme inhibitor (J. Pharm. Sci., 77 (1988), 33-38); a solid solution of insulin in a mixture of stearic acid and a surfactant sold under the trademark "Brij" (J. Pharm. Pharmcol., 33 (1981), 733-734); and the like. However, none of these approaches have been proven satisfactory. They are not suited for application to proteinous drugs in general. Some of these techniques require the use of co-ingredients which have not been established to be pharmaceuticallY acceptable. Others often exhibit undesirable side effects such as vomiting.

JP 61 280 435 discloses a liquid preparation of cyclosporins, comprising in addition to cyclosporins a fatty acid and a nonionic surfactant dispersed in water.

IL 54 041 discloses liquid pharmaceutical compositions for enteral or vaginal application of peptide or proteinous drugs. In particular an insulin containing solution for direct intestinal application is disclosed which is administered by direct injection into the jejunum.

EP-A-0 225 189 discloses an enteric capsule preparation. To prepare the capsule preparation, the active ingredient is dispersed in the first step in an oily carrier together with an absorption promoter. In a second step, the dispersion is encapsuled in conventional gelatine capsule shells. Finally, the capsules are provided with an enteric coating by spraying an enteric coating solution onto the capsules in a rotating coating pan.

By the present invention, there is provided an enteric formulation of physiologically active proteins or peptides (hereinafter referred to as "proteinous drug") in which the active principles are easily absorbed from the intestinal tract and delivered into the blood stream in substantially dose-dependent manner. The present invention utilizes such co-ingredients that have been proven to be pharmaceutically acceptable or inert and, therefore, are those commonly used for the manufacture of various forms of solid medicaments for oral administration. Accordingly, the formulation of the invention is substantially free from any side effect or otherwise adverse action due to the presence of co-ingredients. One of significant advantages of this invention resides in the fact that its application is not limited to a particular class of proteinous drugs but may find use in proteinous principles in general.

Many of proteinous drugs occur as a solution which must be stored in cool, or a lyophilized product which requires subsequent reconstitution in saline or sterile water immediately before use. By the present invention, such drugs may be formulated in a solid medicament for oral administration which is stable for a reasonable period of time and may be administered directly to the patient without the need for reconstitution or other processing. This also achieves transportation and storage advantages over current preparations.

Other objects and advantages of the present invention will become apparent to those skilled in the art as the description proceeds.

### Description of the Invention

According to the present invention, an enteric, pharmaceutical formulation having an enhanced bioavailability is provided comprising a solid mass of an intimate mixture of said proteinous drug, a nontoxic, nonionic surfactant, and a pharmaceutically acceptable, enteric material capable of dissolving in duodenal juice and also in an organic volatile solvent.

The enteric material is a polymeric material used for forming enteric coating on tablets and the like. When the active principle is a peptide or protein which is susceptible to enzymatic degradation in the intestinal tract, an organic acid such as tartaric acid and/or an inhibitor of proteolytic enzymes may be used in combination with the nonionic surfactant so as to suppress the action of proteolytic enzyme. In this case, less than equivalent amount to the organic acid of a carbonate salt such as sodium bicarbonate may be incorporated to give an effervescent formulation.

Preferably, at least 50 %, more preferably not less than 80 % by weight of the entire composition is occupied by the enteric material acting as a carrier. The amount of surfactant may vary with the amount of active principle present in the formulation but must be sufficient to promote the uptake of active principle into the intestinal mucosa while protecting the same from enzymatic degradation in the form of micells.

The formulation of this invention may be prepared using any conventional technique, for example, by dissolving the surfactant and the enteric material in an organic solvent, intimately dispersing the active principle in the solution, evaporating the mixture preferably under vacuum to dryness and pulverizing the resulting mass.

The resulting powder may be administered as such or shaped into granules or other particulate forms. The particulate formulation may be further processed if desired, into tablets, capsules or other unit dosage forms for oral administration.

Alternatively, the enteric material may be present as a discrete coating covering a solid mass of an intimate mixture of other ingredients. Thus, the present invention provides an enteric formulation comprising a solid mass of an intimate mixture of the proteinous drug and a nontoxic, nonionic surfactant, and a coating of the enteric material covering the solid mass, wherein said mixture additionally contains a pharmaceutically acceptable solid organic acid.

When the particulate formulation having a discrete enteric coating is desired, the above procedure may be followed up to the shaping step without initially admixing the enteric material but forming an enteric coating on the shaped particles using any conventional technique.

### Detailed Discussion

A number of physiologically active peptides and proteins are known and find various therapeutic uses. They may be naturally occuring or recombinant products. Physiologically active peptides include not only intact peptides but also their fragments, their family in which one or more constituent amino acids are defect or have been replaced by other amino acids. The term "protein" as used herein also includes glycoproteins.

Non-limiting examples thereof include angiotensin II antagonist peptide, bradykinin, calcitonin-gene associated factor, tissue plasminogen activators, cholecystokinin, delta sleep-inducing peptide (DSIP), β-endorphin, atriopeptin, α-and β-melanocyte-stimulating hormone, nerve peptide, nerve growth factor (NGF), gastrin antagonist peptide, neurotensin, somatostatin, colony-stimulating factors such as G-CSF and GM-CSF, interferon, thymopoietin, tumor necrosis factor, growth hormone, growth hormone-carrier protein complex, human atrial natriuretic peptide (HANP), insulin, luteinizing hormone-releasing factor (LHRH), streptokinase, erythropoietin, urokinase, lysozyme, vasopressin (8-lysine and 8-arginine), oxytocin, desmopressin, cytochome C, histamin-releasing factor, platelet related growth factor human chorionic gonadotropin (HCG), human growth hormone (HGH), adrenocorticotropin (ACTH), calcitonin, parathyroid hormone, Factor XIII, Factor IX, MSH release inhibiting factor, enkephalin, thyrotropin releasing hormone, interleukins such as interleukin-3, renin inhibitor peptides, and the like.

In one of important applications of this invention, the proteinous drug may be cyclosporines, i.e. cyclosporin A, cyclosporin B, cyclosporin C, cyclosporin D and cyclosporin G, which are known to be cyclic oligopeptides having immunosuppressive activity. The term "cyclosporines" as used herein also include those in which one or more amino acids constituting the natural cyclosporines are chemically modified, substituted by other amino acids or removed. Cyclosporines are hardly soluble in water and, therefore oily solutions such as in olive oil have been used for therapeutic application.

A solid, enteric formulation of cyclosporines may be prepared by the present invention with storage, transportation and administration advantages over the oily preparation.

Surfactants are incorporated into the formulation of this invention to serve as an agent for promoting the absortion of protenous drugs by the intestinal tract and also for forming the drug into micells which protect the same from enzymatic degradation. Any nonionic surfactant soluble in a conventional organic solvent such as methanol, ethanol, acetone, ethoxyethanol, methylene chloride and mixtures of these solvents may be used to advantage. Examples thereof include sorbitan fatty acid ester, polyoxyethylenesorbitan fatty acid ester, polyoxyethylene-hydrogenated castor oil, fatty acid alkanolamide, sucrose fatty acid esters, glycerol mono-, di- and trioctanoate. A minor proportion of anionic surfactants such as capric acid or bile salts may be tolerated.

The preparation of this invention contains at least 50 %, preferably not less than 80 % by weight based on the entire composition of an enteric material capable of dissolving in a conventional organic solvent as previously mentioned and also in the duodenal juice. They must be, of course, nontoxic and pharmaceutically acceptable or otherwise have no adverse effect on the active principle. Typically the enteric material is selected from semisynthetic or synthetic polymers having free carboxyl function that have been commonly used for forming an enteric coating on tablets and other orally admistered solid medicaments. Examples thereof include cellulose acetate phthalate, hydroxypropylmethylcellulose succinate, hydroxypropylmethylcellulose phthalate, carboxylmethylethylcellulose, methacrylic acid-methyl methacrylate copolymer, and the like. Optionally incorporated are conventional pharmaceutical excipients such as sugars, e.g. glucose, maltose or sorbitol; organic acids, e.g. tartaric, malonic, maleic, malic, citric or cinnamic; neutral solid materials, e.g. urea; and hydrophilic polymers, e.g. polyethylene glycol, polyvinylpyrrolidone, cyclodextrin, and polyvinyl alcohol. Mixtures of the above-mentioned excipients may be used. For example, the formulations may comprise an organic acid of the above-mentioned class to inhibit the action of proteolytic enzymes with or without combining less than equivalent to the acid of a carbonate salt such as sodium bicarbonate.

The above individual ingredients forming the formulation of this invention are intimately mixed together. By the term "intimately", it is meant that the individual ingredients are not identifiable by the visual inspection of the mixture as in the case of a solid solution. Alternatively, the enteric material may be present, as previously discussed, not in the mixture but as discrete coating on the core of the mixture of other ingredients.

The formulation of this invention may be prepared for example, by dissolving the surfactant and the enteric material in an organic solvent, intimately dispersing the active substance in the solution, evaporating the mixture to dryness, and pulverizing the resulting mass. The above dissolving step may be promoted by stirring. The evaporating step is preferably carried out under vacuum. Although the formulation of this invention may assume a powdered form and administered as such, it may be shaped into granules or other particulate forms as desired. For example, the powdered formulation may be kneaded with a small amount of inert liquid such as water, methanol, ethanol, acetone and the like, and extruding the resulting mass through a mesh screen, and drying the extrudate. Effervescent granules may be prepared by incorporating conventional effervescent agents such as sodium bicarbonate. The granules are provided with an enteric coating thereon using the semi-synthetic or synthetic polymers as previously listed when the nature of carrier material into which the active substance is intimately dispersed is not enteric.

The pharmaceutical preparation of this invention may, of course, comprise other conventional additives such as inhibitors of proteolytic enzymes, coloring agents, flavors, stabilizing agents and the like. Granular or other paticulate preparations of this invention may be further processed into desired unit dosage forms such as tablets, capsules and the like.

The following examples are offered for illustrative purposes only. All parts and percents therein are by weight unless otherwise indicated.

### Example 1

400 mg of L-tartaric acid and 40 mg of polyethylene glycol-hydrogenated castor oil (HCO-60) were dissolved in 5 ml of methanol. This solution was placed in a mortar previously warmed to 30°C. To the solution was added 1.5 mg of recombinant human granulocyte colony-stimulating factor (G-CSF, produced by the method disclosed in PCT Publication WO 87/01132). Immediately after the addition of G-CSF, the mixture was stirred with pestle under hot air current (40°C) and then placed in a desiccator under vacuum overnight to remove the solvent. The resulting solid mass was pulverized with the pestle and kneaded with 30 mg of sodium bicarbonate and a small amount of 70 % ethanol. The mixture was then divided and shaped into pills of about 2 mm size and thoroughly dried. The dried pills were given a coating of hydroxypropylmethylcellulose phthalate (HP-55) to obtain an enteric formulation.

The above procedure was repeated without incorporating sodium bicarbonate to obtain a similar formulation.

### Example 2

A solution of 900 mg of HP-55 and 200 mg of HCO-60 in 2 ml of a 3:10 mixture of methanol and methylene chloride was poured into a vial containing 1.5 mg of lyophilized G-CSF and stirred until the mixture became a solution. This solution was cast into a Teflon® tubing segment having a diameter of 3 mm and allowed to stand at room temperature for five hours. Then the tubing segment was placed in a desiccator under vacuum to remove the solvent completely. The resulting solid mass was withdrawn from the Teflon® mold and cut into segments of about 2 mm length.

### Example 3

25 mg of insulin, 400 mg of L-tartaric acid and 40 mg of HCO-60 were dissolved in 5 ml of a 8:2 mixture of methanol and 0.1 N HCl. This solution was placed in a mortar previously warmed to 30 °C, stirred with a pestle under hot air current (40°C), then evaporated in a desiccator under vacuum overnight to remove the solvent and pulverized with the pestle. The resulting powder was shaped into granular form with a small amount of 80 % ethanol as in Example 1 and processed with HP-55 to give an enteric formulation.

### Example 4

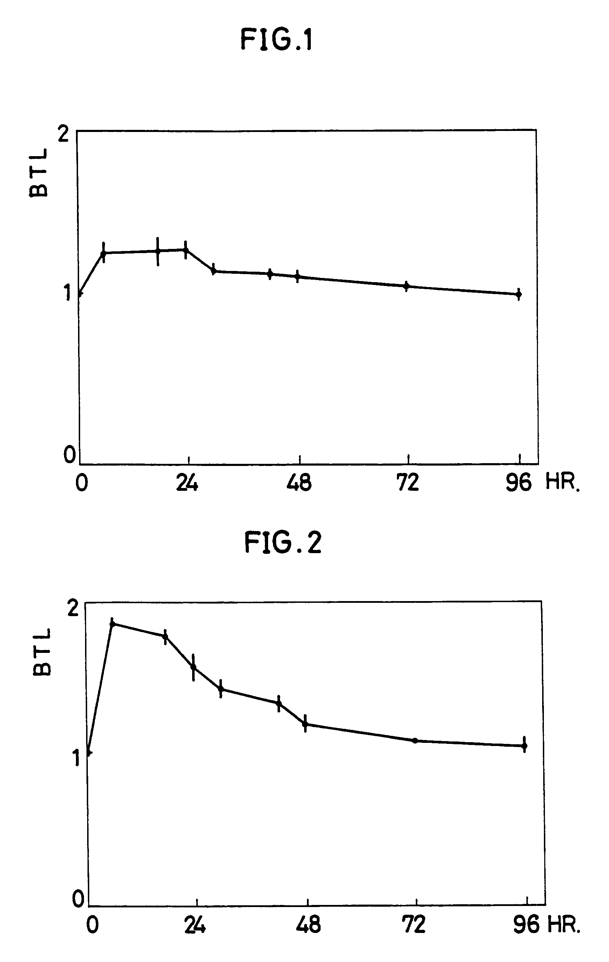

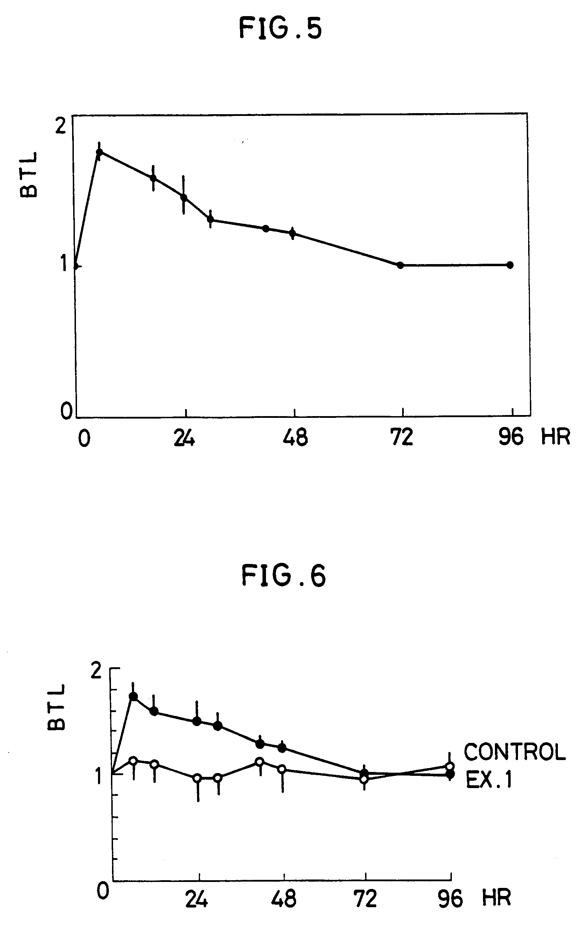

Three male Wistar rats, weighing 300-400 g were used in each experiment. The rats were given a dose of the enteric preparation of Example 1 corresponding to 1.5, 0.75 and 0.375 mg/kg of G-CSF, respectively, by administering through an incision on the stomach into the duodenum. The control group was given a placebo preparation which was free of G-CSF but otherwise identical to the preparation of Example 1. Blood samples were taken from the tail artery before the administration of the test preparations and subsequent to the administration over 4 days. The blood total leucocyte (BTL) counts were determined and expressed as the relative value, which was obtained by dividing the BTL count by the respective control BTL count. The results obtained are shown in the accompanying drawings, in which Figs. 1, 2, 3 and 4 illustrate BTL dynamics obtained by the administration of the effervescent preparation of Example 1 at a G-CSF dose level of zero (control), 1.5 mg/kg, 0.75 mg/kg and 0.375 mg/kg, respectively. Fig. 5 shows similar dynamics of the group given the non-effervescent preparation of Example 1 at a G-CSF dose level of 1.5 mg/kg. Fig. 6 is a BTL dynamic curve similar to Fig. 2 curve (1.5 mg G-CSF/kg) in comparison with the control over 96 hours after administration.

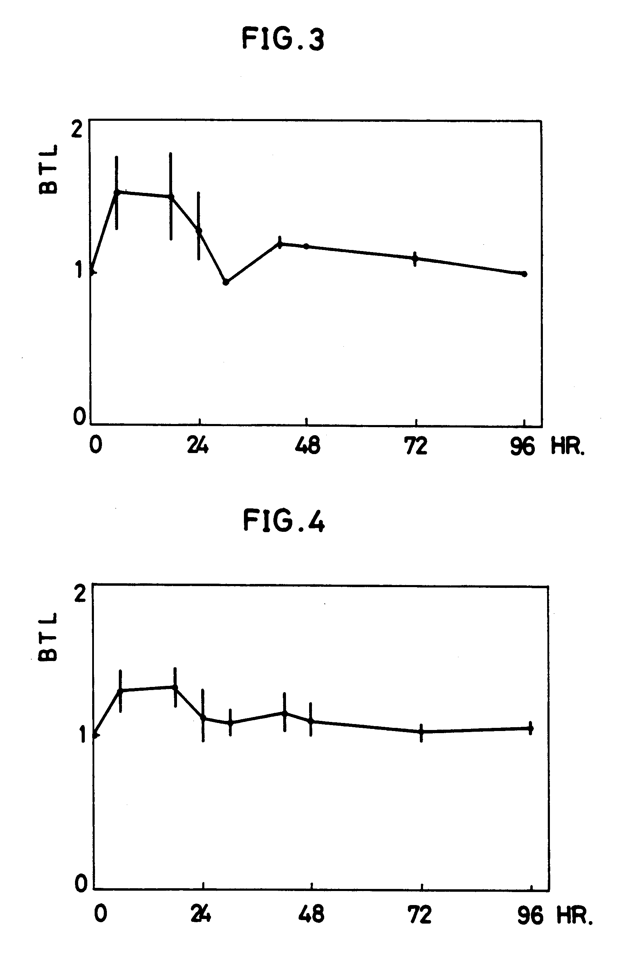

As can be seen in the drawings, a significant increase in the BTL count was observed over 48 hours at the G-CSF dose of 1.5 mg/kg compared with the control group. The BTL dynamics curves shown in Figs. 1-4 indicate that the increase in BTL count is of dose-dependency nature, while no significant difference may be seen in the curve of Fig. 4 (0.357 mg/kg) as compared with the control (Fig. 1). The non-effervescent preparation of Example 1 was also substantially as effective as the effervescent preparation in the increase of BTL count as demonstrated in Fig. 5.

### Example 5

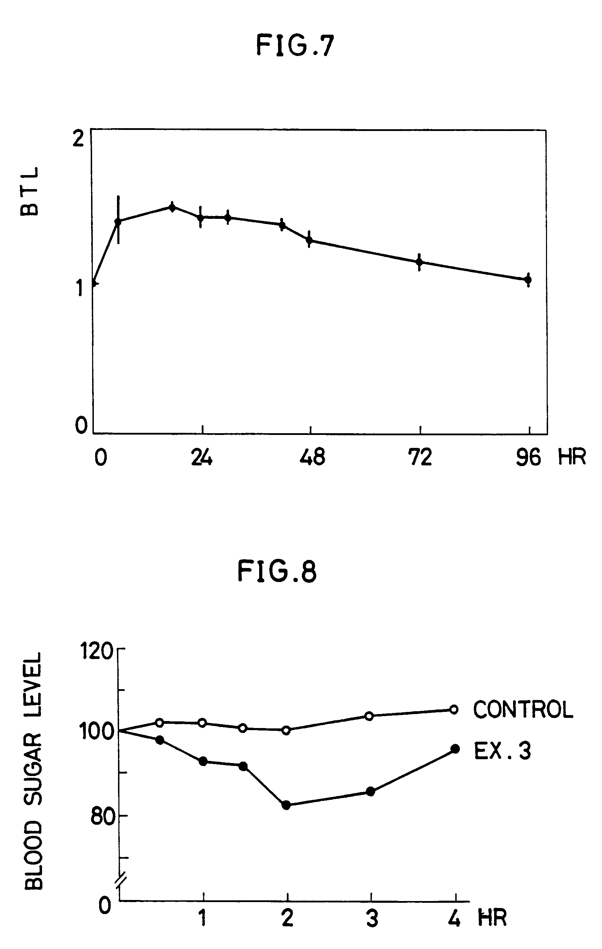

The experiments of Example 4 were repeated using the enteric preparation of Example 2 containing no L-tartaric acid. The BTL dynamics curve at a G-CSF dose level of 1.5 mg/kg is shown in Fig. 7. The increase in BTL level in Fig. 7 is not remarkable as Fig. 2, but still significant compared to the control shown in Fig. 1.

### Example 6

Analogous to Example 4, the enteric preparation of insulin produced in Example 3 was administered to the rat at a dose level of 2.5 mg/rat as insulin. As a control, another rat was given a placebo preparation which was free of insulin but otherwise identical to the preparation of Example 3. Blood samples were taken from the jugular vein before and after the administration of the test preparations over 4 hours. The dynamics of blood sugar levels as expressed as the index relative to the control are shown in Fig. 8. The blood sugar level decreased to nearly 80 % of the control.

### Example 7

500 mg of a renin inhibitor peptide having the following formula: 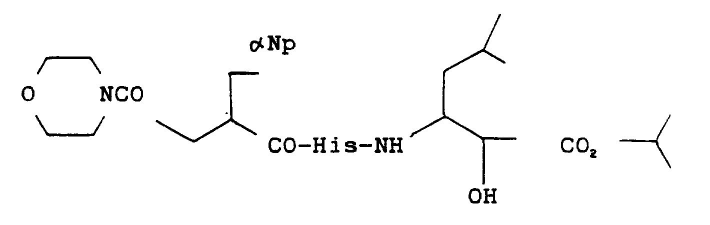
M.W.=649.79, C₃₅H₄₇N₅O₇, 100 mg of HCO-60 and 4.2 g of L-tartaric acid were dissolved in 10 ml of methanol at 40 °C. This solution was placed in a mortar, stirred with a pestle under hot air current, placed in a desiccator under vacuum to remove the solvent and pulverized with the pestle. After the addition of 200 mg of sodium bicarbonate, the mixed powder was kneaded with a small amount of methanol, extruded through a 14 mesh wire screen and dried to give granules which were then given an enteric coating of HP-55.

Analogous to Example 4, the above enteric preparation was administered to the rats at a dose level of 10 mg/rat as the renin inhibitor. As a control, another group of rats was given a drug suspended in CMC solution at the same dose level.

Blood samles were taken from the carotid artery after the administration and assayed for the concentration of the renin inhibitor using HPLC.

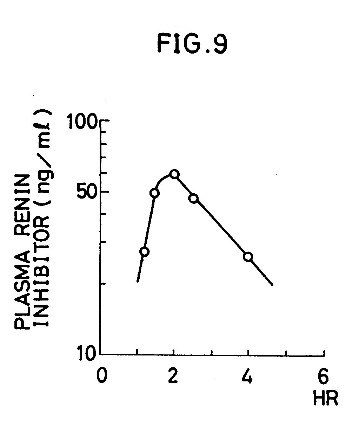

The concentration was less than the detection limit of 25 ng/ml for the CMC suspension whereas the concentration increased to a maximum of 60 ng/ml for the formulation of this invention as shown in Fig. 9.

## Claims

1. An enteric pharmaceutical formulation comprising a solid mass of an intimate mixture of a physiologically active peptide or protein excluding cyclosporins, a nontoxic nonionic surfactant, and a pharmaceutically acceptable, enteric material capable of dissolving in duodenal juice and also in an organic volatile solvent.

2. The pharmaceutical formulation of Claim 1, wherein said intimate mixture additionally contains a pharmaceutically acceptable solid organic acid.

3. The pharmaceutical formulation of Claim 2 including an amount of sodium bicarbonate less than equivalent to the organic acid

4. The pharmaceutical formulation of claim 1, wherein said intimate mixture additionally contains an excipient selected from the group consisting of sugars, neutral solid materials and hydrophilic polymers.

5. The pharmaceutical formulation of Claim 1, wherein said peptide or protein is insulin, human granulocyte colony-stimulating factor, or a renin inhibitor peptide.

6. The pharmaceutical formulation of Claim 2, wherein said organic acid is L-tartaric acid.

7. The pharmaceutical formulation of Claim 1, wherein said formulation is shaped into granules or other particulate forms.

8. The pharmaceutical formulation of Claim 7, wherein said formulation is further processed into tablets, capsules or other solid unit dosage forms.

9. An enteric pharmaceutical formulation comprising a solid mass of an intimate mixture of a physiologically active peptide or protein excluding cyclosporins with a nontoxic nonionic surfactant, and a discrete enteric coating covering the core of said solid mass, wherein said intimate mixture additionally contains a pharmaceutically acceptable solid organic acid.

10. The pharmaceutical formulation of Claim 9, wherein said core of said solid mass additionally contains an amount of sodium bicarbonate less than equivalent to the organic acid.

11. The pharmaceutical formulation of Claim 9, wherein said intimate mixture additionally contains an excipient selected from the group consisting of sugars, neutral solid materials and hydrophilic polymers.

12. The pharmaceutical formulation of Claim 9, wherein said peptide or protein is insulin, human granulocyte colony-stimulating factor, or a renin inhibitor peptide.

13. The pharmaceutical formulation of Claim 9, wherein said organic acid is L-tartaric acid.

14. The pharmaceutical formulation of Claim 9, wherein said formulation is shaped into granules or other particulate forms.

15. The pharmaceutical formulation of Claim 9, wherein said formulaion is further processed into tablets, capsules or other solid unit dosage forms.

## Patentansprüche

1. Enterische pharmazeutische Formulierung, umfassend eine feste Masse aus einem innigen Gemisch eines physiologisch aktiven Peptides oder Proteins, wobei Cyclosporine ausgenommen sind, mit einem nichttoxischen, nichtionischen, oberflächenaktiven Mittel und einem pharmazeutisch verträglichen enterischen Material, das sowohl in Duodenalsaft als auch in einem flüchtigen organischen Lösungsmittel löslich ist.

2. Pharmazeutische Formulierung gemäß Anspruch 1, worin das innige Gemisch zusätzlich eine pharmazeutisch verträgliche, feste organische Säure enthält.

3. Pharmazeutische Formulierung gemäß Anspruch 2, die Natriumbicarbonat in einer Menge aufweist, die geringer ist als die äquivalente Menge an organischer Säure.

4. Pharmazeutische Formulierung gemäß Anspruch 1, worin das innige Gemisch zusätzlich einen Exzipienten enthält, der ausgewählt ist unter Zuckern, neutralen Feststoffen und hydrophilen Polymeren.

5. Pharmazeutische Formulierung gemäß Anspruch 1, worin das Peptid oder Protein Insulin, menschlicher Granulozytenkolonie-stimulierender Faktor oder ein Renininhibitorpeptid ist.

6. Pharmazeutische Formulierung gemäß Anspruch 2, worin die feste Säure L-Weinsäure ist.

7. Pharmazeutische Formulierung gemäß Anspruch 1, worin das Mittel als Granulat oder in anderer Teilchenform vorliegt.

8. Pharmazeutische Formulierung gemäß Anspruch 7, worin das Mittel zu Tabletten, Kapseln oder anderen festen Dosierungsformen weiterverarbeitet ist.

9. Enterische pharmazeutische Formulierung, umfassend eine feste Masse aus einem innigen Gemisch eines physiologisch aktiven Peptids oder Proteins, wobei Cyclosporine ausgenommen sind, mit einem nichttoxischen, nichtionischen, oberflächenaktiven Mittel, und einer diskreten enterischen Beschichtung, die den Kern der festen Masse umschließt, wobei das innige Gemisch zusätzlich eine pharmazeutisch verträgliche, feste organische Säure enthält.

10. Pharmazeutische Formulierung gemäß Anspruch 9, worin der Kern der festen Masse zusätzlich Natriumbicarbonat in einer Menge enthält, die geringer ist als die äquivalente Menge an organischer Säure.

11. Pharmazeutische Formulierung gemäß Anspruch 9, worin das innige Gemisch zusätzlich einen Exzipienten enthält, der ausgewählt ist unter Zuckern, neutralen Feststoffen und hydrophilen Polymeren.

12. Pharmazeutische Formulierung gemäß Anspruch 9, worin das Peptid oder Protein Insulin, menschlicher Granulozytenkolonie-stimulierender Faktor oder ein Renininhibitorpeptid ist.

13. Pharmazeutische Formulierung gemäß Anspruch 9, worin die organische Säure L-Weinsäure ist.

14. Pharmazeutische Formulierung gemäß Anspruch 9, worin das Mittel als Granulat oder in anderer Teilchenform vorliegt.

15. Pharmazeutische Formulierung gemäß Anspruch 9, worin das Mittel zu Tabletten, Kapseln oder anderen festen Dosierungsformen weiterverarbeitet ist.

## Revendications

1. Formule pharmaceutique à délitage intestinal comprenant une masse solide d'un mélange intime d'un peptide ou d'une protéine physiologiquement actifs à l'exclusion des cyclosporines, un agent tensioactif non-ionique non-toxique et une matière à délitage intestinal pharmaceutiquement acceptable, capable de se dissoudre dans le suc duodénal et aussi dans un solvant organique volatil.

2. Formule pharmaceutique selon la revendication 1, dans laquelle ce mélange intime contient en outre un acide organique solide pharmaceutiquement acceptable.

3. Formule pharmaceutique selon la revendication 2, contenant une quantité de bicarbonate de sodium inférieure à celle équivalant à l'acide organique.

4. Formule pharmaceutique selon la revendication 1, dans laquelle ce mélange intime contient en outre un excipient choisi parmi des sucres, des matières solides neutres et des polymères hydrophiles.

5. Formule pharmaceutique selon la revendication 1, dans laquelle ce peptide ou cette protéine est l'insuline, le facteur stimulant les colonies de granulocytes humains ou un peptide inhibiteur de la rénine.

6. Formule pharmaceutique selon la revendication 2, dans laquelle cet acide organique est l'acide L-tartrique.

7. Formule pharmaceutique selon la revendication 1, dans laquelle cette formule est façonnée en granulés ou en d'autres formes particulaires.

8. Formule pharmaceutique selon la revendication 7, dans laquelle cette formule est en outre transformée en comprimés, en capsules ou en autres formes thérapeutiques unitaires solides.

9. Formule pharmaceutique à délitage intestinal comprenant une masse solide d'un mélange intime d'un peptide ou d'une protéine physiologiquement actifs à l'exclusion des cyclosporines avec un agent tensioactif non-ionique non-toxique, et un enrobage à délitage intestinal distinct recouvrant le noyau de cette masse solide, dans laquelle ce mélange intime contient en outre un acide organique solide pharmaceutiquement acceptable.

10. Formule pharmaceutique selon la revendication 9, dans laquelle ce noyau de cette masse solide contient en outre une quantité de bicarbonate de sodium inférieure à celle équivalant à l'acide organique.

11. Formule pharmaceutique selon la revendication 9, dans laquelle ce mélange intime contient en outre un excipient choisi parmi des sucres, des matières solides neutres et des polymères hydrophiles.

12. Formule pharmaceutique selon la revendication 9, dans laquelle ce peptide ou cette protéine est l'insuline, le facteur de stimulation des colonies de granulocytes humains ou un peptide inhibiteur de la rénine.

13. Formule pharmaceutique selon la revendication 9, dans laquelle cet acide organique est l'acide L-tartrique.

14. Formule pharmaceutique selon la revendication 9, dans laquelle cette formule est façonnée en granulés ou en d'autres formes particulaires.

15. Formule pharmaceutique selon la revendication 9, dans laquelle cette formule est en outre transformée en comprimés, en capsules ou en d'autres formes thérapeutiques unitaires solides.
